# EUROPEAN PATENT APPLICATION

(11) **EP 1 850 289 A1**
(43) Date of publication of application: **31.10.2007**
(21) Application number: 05822640.8
(22) Date of filing: 19.12.2005
(51) Int. Cl.: G06Q 50/00, G08B 13/00

(54) **APPLICATION AND SYSTEM FOR RFID IN WORKFLOW OF BLOOD CENTER**

(30) Priority: 19.01.2005 CN 200510038156
(71) Applicant: Chen, Yan, Nanjing, Jiangsu 210016 (CN)
(72) Inventor: Chen, Yan, Nanjing, Jiangsu 210016 (CN)
(74) Representative: Isenbruck, Günter
(86) International application number: PCT/CN2005/002236
(87) International publication number: WO 2006/076848

(57) **Abstract**

An application and a system of RFID (Radio Frequency Identification) in the workflow of a blood center and a medical institution from a network Customer Relationship Management (CRM) information system. The CRM comprises an RFID technique system, a computer database system, and a computer information network. The information transferred between an integrated circuit and a reader-writer occurs through the radio waves in the workflow of, for example, a blood center or other medical institution. The related information ranges from identity information to biological product information. In each procedure of the blood collecting and supply workflow, the information is read/written by the computer into electronic tag and through the computer information network into the service management information system. The work flow of blood centers (stations), such as blood test reports before and after blood transfusions and the desired information of people and objects involved in blood transfusions, can thereby be recorded completely, integrally, precisely, and comprehensively by the RFID system.

## Description

### Field of the Invention

The present invention introduces a nationwide Customer Relationship Management (CRM) network comprising Radio Frequency Identification (RFID) technique, computer database technique, and computer network technique into the medical field, and particularly relates to the application of RFID in workflow of blood centers and medical institutions as well as an RFID system.

### Background of the Invention

Some medical tests are not effective in real time. For example, there is "window period" problem in detection of hepatitis C due to specificity of hepatitis C virus; therefore, not all patients carrying hepatitis C virus around the world can be detected with existing reagents and detection methods. During the procedure of blood donation, it would be helpful for the medical staff to decrease the "leakage rate" if they can obtain the donor's information through an information channel.

When a medical institution handles blood donations, the medical institution usually carries back the blood sample to the laboratory for testing. If the blood sample fails the test, the blood has to be discarded, and the one-off containers used to transfer the blood from the collection site to the fixed service location of the medical institution has to be discarded with the blood. That results in waste in many aspects. Such wastes can be prevented if the donor's information can be obtained in advance through an information channel before the blood collection.

At present, documents such as paper blood donation certificates are susceptible to forgery, and are often forged or resold.

In today's blood transfusion and bone marrow transplantation operations, patients can't obtain the quality information of the blood or blood products transfused. If a patient contracts a disease or infection after medical treatment, it can be difficult to provide evidence on whether the problem occurs in the curative preparation-and-test link or in the actual medical treatment link and thereby ascertain the liabilities among the hospital that carried out the medical treatment, the blood center that prepared the curative, and the bone marrow donors.

Barcodes are also susceptible to forgery and possibly become ineffective if they are stained; in addition, the operator has to operate carefully when he/she reads the barcodes. One dimensional barcodes can hardly store any other information except for identification character strings. Even two-dimensional barcodes may become ineffective after they are stained and have not enough storage capacity. In addition, they don't support remote measurement or real-time traceability. Though conventional two-dimensional barcodes can store more information than one dimensional barcodes, they are exposed and may become ineffective if they are stained. If there is demand for forgery prevention (e.g., data signature and compressed storage of retina information) or encryption for two dimensional barcodes, the storage capacity will not meet the challenge. Furthermore, two-dimensional barcodes can't even meet the confidentiality requirement that they can't read it automatically.

Radio frequency identification (RFID) technique has been developed greatly; RFID tags are active or passive tags composed of coupling elements and chipset; each tag has a unique electronic ID. TI Company (USA) and INSIDE Company (France) are major manufacturers of high-capacity encrypted electronic tags.

### Summary of the Invention

The object of the present invention is to apply RFID technique, computer information networks, and computer database techniques for CRM in the medical domain. Particularly, the present invention puts forward a method and a system of using RFID in the workflow of blood centers and medical institutions. The present invention also puts forth a blood identification device, and particularly an automatic blood identification system, that has network service, support, and transmission capabilities for blood centers.

The present invention employs handheld or fixed type readers/writers in the RFID system, wherein, the terminal equipment is connected to the reader/writer through a data transmission bus, the antenna of the reader/writer sends signals at a preset frequency, the RFID tag enters it produces induced current and thereby obtains energy and sends its coding information and where the RFID tag enters into the magnetic field the reader/writer reads the information, decodes it, and then sends the decoded information to the terminal equipment for data exchange. The blood centers and medical institutions include, for example blood stations at all levels, clinical laboratories in hospitals, the China Marrow Donor Program, sperm banks, ovum banks, and other medical institutions that have electronic tags attached to things (e.g., blood collection bags) and files (e.g., blood donation certificates), employ data encryption, and carry out identity authentication or access authority control for the person who accesses the information. In addition, the present invention provides technical security control methods and means.

An object of the present invention is to provide a system for using RFID in the workflow of blood centers and medical institutions comprising a nationwide network of Customer Relationship Management (CRM) system (e.g., a blood information management system) composed of a Radio Frequency Identification (RFID) system, computer database system, and a computer information network. The information transfer between the integrated circuits in electronic tags attached to biological products (e.g., blood) or files (i.e., electronic tags attached to things or files) is through radio waves.

In many procedures of the workflow of the medical institution, relevant information is written into the electronic tag attached to an object or file by the person responsible for the procedure by means of a computer equipped with an electronic tag reader/writer, and is written into the service management information system through the computer information network. The computer information network can be implemented with existing public lines or wireless data links, or private lines or wireless data links. In addition, data-encryption techniques can be employed as required.

These electronic tags may have stored data-encryption capability and/or the capability of identity authentication (e.g., requesting that a password be provided) and access-authority control for the person who accesses the information. In addition, data-encryption techniques can be employed as required.

Useful information in the service-handling process is stored and a variety of categorized, sorted, and statistical information is created by means of the RFID system and other information acquisition means, and a variety of information-search, service-management, and control functions are provided. The system can carry out identity authentication and access-authority control for the person who accesses the information, and provide corresponding information in accordance with the person's privilege.

The present invention employs devices and systems that execute special functions and can be controlled by the inner computers. the present invention preferably does not utilize general-purpose computers or run solidified software (i.e., firmware), which can't be or is difficult to be modified by the terminal user. The "invisibility" feature differentiates embedded computers (terminals) from general-purpose PCs.

### Application of RFID tags:

The RFID tags can be used to log all workflow of the blood center. Such workflow may include, for example, collecting blood from the source (blood donors), sub-packing components for test, testing (including initial testing and re-testing), transporting the acceptable blood to blood storeroom (stored in ice room or discarded), transporting the blood to the blood bank (clinical laboratory) in a hospital and matching blood type, transfusing the blood to a donee, and logging the destination of blood bags. The electronic tags feature easy operation, contactless read/write functionality, high storage capacity, high encryption, and forgery-prevention capabilities, among other benefits. These techniques are derived from electronic computer technology - a variety of computer software and hardware technology can be used for electronic tags flexibly as required. Presently, the market price of electronic tags in affordable to many applications.

Blood centers (such as blood stations), medical institutions such as hospitals, bone marrow banks, sperm banks, and ovum banks have similar workflows - collection, testing, processing, storage, transporting to medical treatment units, and then medical treatments. In this workflow, there are many things and files to be managed. On one hand, the institutions and their administrative units must carry out process management and quality control to ensure that national standards and regulations are strictly followed; on the other hand, the end users (patients) have the right to obtain the above information. There are also certain confidentiality requirements for this information in above process. For example, in the same medical institution, the person responsible for each service can only control information related to his/her service, only certain information can be shared and between different medical institutions patients only have the right to know the quality information on the curative applied to them and can't know other confidential information, such as the donors. With computer information network technology, the information can be accessed, searched, summarized, and processed as required at any time in any place.

The embedded electronic recognition system of objects (e.g., blood bags) and files (e.g., blood donation certificates) in medical institutions such as blood centers or blood stations, hospitals, bone marrow banks, sperm banks, and ovum banks, etc. comprises Radio Frequency Identification (RFID) system, database computer system, and computer information network. These systems constitute a nationwide network CRM information system, such as blood information management system. Through the computer information network, the information stored in the electronic tags attached to biological products (e.g., blood) and files is transmitted. In addition, the information is protected for confidentiality and forgery prevention.

The RFID system for workflow in blood centers and medical institutions provided in the present invention employs embedded electronic recognition technology to implement a network management system for automatic identification (medical or blood CRM) in medical institutions such as blood centers. The system comprises terminal equipment (or management server), a blood collection vehicle (or blood stations or plasma stations), blood donation certificates, and RFID devices, wherein the RFID devices include electronic tag readers/writers, antennae, electronic tags that have unique electronic ID and are attached to blood bags to identify the target object, and electronic blood donation certificates containing a variety of information and the reader/writer is a handheld or fixed type working at 13.56MHz and utilizes a computer information network and computer database technology for customer information management.

Said terminal equipment is connected to the reader/writer through a data bus; the antenna of the reader/writer sends signals at preset frequency; when the RFID tag enters into the magnetic field, it produces induced current and thereby obtains energy and sends its coding information; the information is received by the reader/writer, decoded, and then sent to the terminal equipment for data exchange; in that way, the RFID tags that have unique electronic ID and are attached to the blood bags or RFID blood donation certificates that contain a variety of information can be identified.

During the work, an electronic tag is attached to the blood bag first; the information of the electronic tag is written by the reader/writer into the corresponding terminal equipment; the blood donor obtains an RFID blood donation certificate; said RFID blood donation certificate is identified and checked by an integrated identifier, and the information is sent to the management server; and the management server reads the RFID tag via the remote reader/writer to keep track of the blood donation certificate.

The electronic tags work with the computer information system via terminal equipment and RFID system equipment; said RFID system equipment comprises an antenna; said electronic tag reader/writer is a handheld or fixed type, working at 125KHz-134.2KHz (low-frequency) and 13.56MHz (high frequency). Said terminal equipment and electronic tag reader/writer are deployed in the computer equipment room, blood storeroom, or at the blood-release window in a blood center, or in the blood storeroom or clinical laboratory in a hospital.

Said RFID blood donation certificate has an identifier for special blood types and blood from RH-Negative blood donors. The computer with the electronic tag reader/writer is connected to the computer information network through an existing public line or wireless data link, or private line or wireless data link, and can employ a data-encryption technique.

There, may be confidentiality requirement, for the above information. For example, in the same medical institution, the person responsible for each service can only control information related to his/her service; only certain information can be shared among different medical institutions; patients only have the right to know the quality information on the curative applied to them and can't know other confidential information, such as the donors.

RFID technique comes from modem electronic computer technology. In fact, an electronic tag is often a low-cost ultra-micro computer system with complete structure and function, intangible to the user. Electronic computer software and hardware techniques can be applied in the electronic tag. This means the typical password control and access authority control functions available in computer systems can also be implemented in the small electronic tag.

A feature of the electronic tag is that the person who wants to access the electronic tag must submit the password before he/she can read/write the information in the electronic tag; otherwise the password stored in the electronic tag can't be read out. The password verification operations are completed entirely in the electronic tag.

Another feature of the electronic tag is that it can contain several storage pages, each of which can be set with a separate password. The information in the page can be accessed only after the corresponding password for the storage page is submitted.

Another feature of the electronic tag is that it can be set to "read only" permanently and non-reversibly after all "write" operations are completed. After the electronic tag is set to permanent "read only" no information can be written into it and no information contained in it can be modified (even if the correct password is submitted); instead, only the information written into it before it is set to permanent "read only" can be read out.

Another feature of the electronic tag is that if an intruder attempts to bypass the password control and read/write information from/into the electronic tag, he/she has to open the encapsulation of the electronic tag. Since the electronic tag contains precise integrated circuits and the integrated circuit core is bonded with the encapsulation tightly, once the intruder attempts to open the encapsulation of the electronic tag, the integrated circuit core will be damaged completely and will be detected by the administrator. As a result, illegal read/write operations can be deferred.

It can be seen from these features that the persons responsible for the procedures in the same medical institution and the persons between medical institutions can access specific information in the electronic tag with proprietary passwords, but can't access any information that is not permitted with the access right. Also, after the patient obtains the electronic tag, since he/she doesn't know the password to access the tag, he/she will be unable to read any information without the permission (password) and specific reader/writer, even though all information is in his/her hand. Also, after the entire workflow is completed, the electronic tag attached to the curative can be set to "read only" permanently and non-reversibly so that a person can't modify the content in the electronic tag. In case of any accident, the actual historical truth can be reflected by reading the information stored in the tag, and thereby powerful evidence can be provided to ascertain culpability. Also, if the electronic tag is attached to a blood donation certificate, it can be verified by checking the password. Only the medical institution that has the password can write information (e.g., add a donation record) to the blood donation certificate.

In addition, when accessing the electronic tag, the reader can be at a certain distance from the tag, or even separated from the tag by non-conductive objects. This differs from the use of barcodes, in which the operator has to align the barcode accurately to the barcode reader/writer. In contrast, an operator can access the electronic tag easily through the RFID technique, thereby alleviating the operator labor intensity.

The RFID technique supports parallel recognition (i.e., an electronic tag reader/writer can be used to access more than one electronic tag at the same time). For example, a tailored large electronic tag reader/writer can be deployed at the storeroom door to access all electronic tags on multiple items on a vehicle. This can simplify the counting work greatly, and differs from the conventional barcode technique.

### Combination of electronic tag and computer information system:

Though electronic tags have advantages in security and convenience, computer information network and computer database system are required for the administrators to learn about the macroscopic information (mainly some statistical information) and implement information-sharing between the medical institutions under certain rules.

The electronic tags can be cross-checked with the data items in the computer information network and the computer database system so as to further enhance forgery-prevention performance. In addition, normalized data storage in the computer database system provides a necessary basis for information-sharing between the medical institution and the administrative organization, as well as between medical institutions under certain rules.

An advantage of the present invention is to "stop" or "block" patients carrying or infected by diseases such as AIDS, hepatitis C, or syphilis virus, and help government to control, treat, or suppress infection and propagation of these diseases among uninfected people, and assist with the handling of infectious-disease outbursts and natural and man-made disasters, and carry out nationwide supervision and control and field instruction. After RFID technique is applied to blood donation certificates, all persons who are detected to have AIDS or hepatic C virus will be prohibited from donating blood all their life; the prohibition ordnance can be distributed through the nationwide network. Whenever such a person gives blood at any blood collection point later, he/she can be identified and rejected automatically by the system. The system thereby provides a platform for the control, suppression, and prevention of AIDS and other infectious diseases among uninfected people and greatly enhances the ability to provide nationwide instruction at any time.

In addition, the present invention can reduce "leakage rate" and lawsuits resulting from blood transfusion accidents, reduce "false positive" or "false negative" results in sample tests, and thereby decrease the probability of transmitting AIDS and other infectious diseases during blood transfusions. The present invention can also enhance safety and a sense of trust of individuals, such as doctors and patients, to the important medical means (such as blood transfusions), and is helpful tin the clinical application of new medical techniques.

The present invention can also save energy and costs since it is unnecessary to store a blood donor's blood samples permanently for future reference. That approach can eliminate the traditional practice that additional 10-20ml blood must be taken from the blood donor in each blood donation process, and it also provides better legal evidence, if necessary.

The RFID system records the workflow in blood centers (blood stations) completely, integrally, precisely, comprehensively blood test reports before and after blood transfusion and the information of people and objects involved in blood transfusions. The RFID system can also facilitate the ability of health officials to view and provide instructions around the country and provide effective and legal medical evidence for lawsuits related to disputes in blood transfusion. Since the event is recorded by three workers on different posts at different locations and the workers are even unknown to each other, the reliability and authenticity of the records is improved greatly. The records can be reviewed conveniently, and can be stored permanently; in addition, the energy consumption and blood transfusion fund are reduced greatly. The workload and labor intensity of the blood collection workers are alleviated, and the sense of responsibility is enhanced.

The advantages and characteristics of the present invention will be illustrated and described but not limited in the following preferred embodiments, with reference to the accompanying drawings.

### Brief Description of the Drawings

Fig. 1 is a schematic diagram of the RFID technique according to an embodiment of the present invention;
Fig. 2 is a structural schematic diagram of the system according to an embodiment of the present invention;
Fig. 3 is a schematic diagram of tracking blood donation certificate according to an embodiment of the present invention;
Fig. 4 is a schematic diagram of the RFID network management system of nation-wide blood center according to an embodiment of the present invention.

While the present invention is amendable to various modifications and alternative forms, specifics thereof have been shown by way of example in the drawings and will be described in detail. It should be understood, however, that the intention is not to limit the present invention to the particular embodiments described. On the contrary, the intention is to cover all modifications, equivalents, and alternatives falling within the spirit and scope of the present invention as defined by the appended claims.

### Detailed Description of the Embodiments

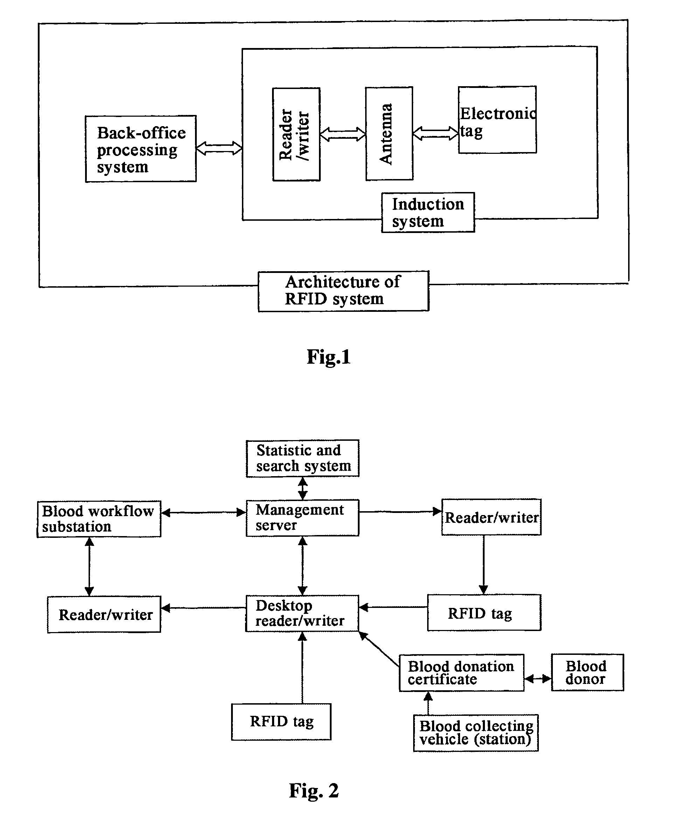

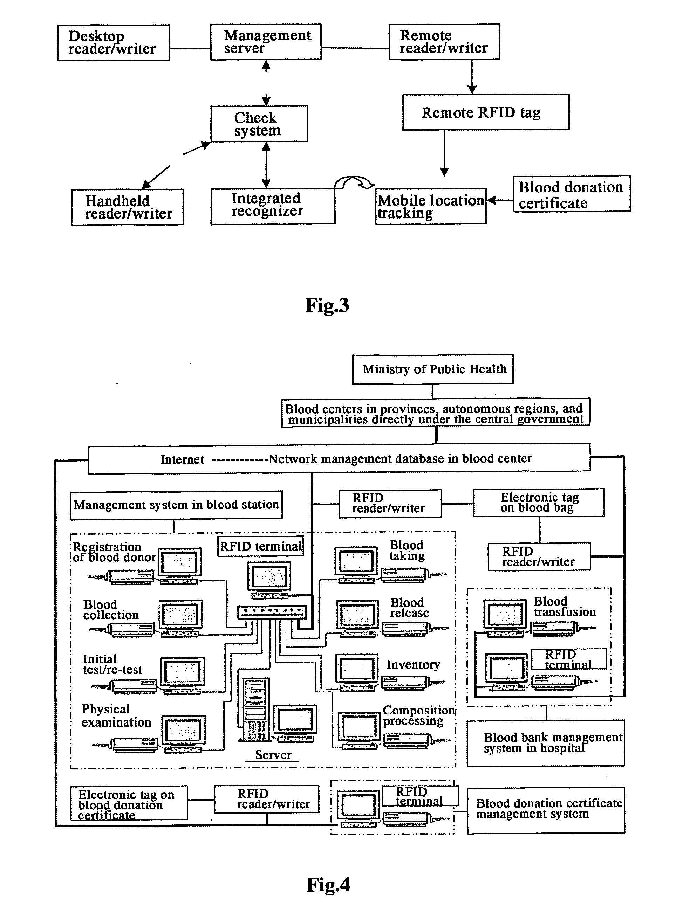

Some embodiments of the method and system of the present invention in the blood management field are shown in Figs. 1-4. The technical scheme of the present invention employs embedded RFID technique to implement a RFID network management system (medical blood CRM) of blood center.

Referring to Figs. 1-2, the system of the present invention comprises terminal equipment (or management server), blood collection Vehicle (or blood stations or plasma stations), blood donation certificates, RFID devices including electronic tag reader/writers, antennae, electronic tags that have unique electronic ID and are attached to blood bags to identify the target object, electronic blood donation certificates containing a variety of information, and computer information network and computer database technology for customer information management. Reader/writer is of handheld or fixed type working at 13.56MHz (high frequency).

The terminal equipment is connected to reader/writer through a data bus. Antenna of reader/writer sends signals at a preset frequency. When RFID tag enters into a magnetic field, it produces induced current and thereby obtains energy and sends its coding information Reader/writer reads the information, decodes it, and then sends the decoded information to the terminal equipment for data exchange.

During the work, electronic tag is attached to a blood bag first. The information of electronic tag is written by reader/writer into the corresponding terminal equipment. Blood donor obtains RFID blood donation certificate. RFID blood donation certificate is identified and checked by an integrated identifier, and the information is sent to management server. Management server reads RFID tag via remote reader/writer 18 to keep track of blood donation certificate.

The information recorded by the present invention can include blood collection and supply information of blood center and blood transfusion process and blood sample information, nationwide network information, forgery-prevention and tracking information of blood donation certificate 16, and customer relationship management (CRM) information.

The RFID system equipment generally comprises RFID tags. RFID tags can be passive tags composed of coupling elements and a chipset. Each RFID tag has a unique electronic ID and can be attached to a blood bag to identify the target object. In the initial stage of an example embodiment, two types of electronic tags different in storage capacity will be used.

The first type is a 32KBit encrypted electronic tag, such as, for example, from INSIDE Company (France), with 4000-bytes storage capacity (3200 bytes are used in the software design for the present invention - about 2400 bytes for software, about 800 bytes for retina recognition, and the remaining bytes are reserved).

The second type is a 2KBit encrypted electronic tag from TI Company (USA).

RFID tags are used to log all workflow of the blood center. This workflow can include collecting blood from the source (blood donors), sub-packing components for testing, testing (including initial testing and re-testing) transporting acceptable blood to a blood storeroom (where the blood can be stored in an ice room or discarded), transporting the blood to a blood bank (clinical laboratory) in a hospital matching blood type, transfusing the blood in to a donee, and logging the destination of blood bags.

All procedures are recorded. All people participating in the blood process are also recorded. This information can time, location, name of the blood collection and supply unit, name of the blood using unit (hospital), identity card number, duty, blood type requested, approved, and transfused, quantity of blood transfused (ml), and signatures of the operator and department directors in each link among other information.

In an example embodiment, the present invention accurately records the blood test report of the donee (patient) provided by the hospital before a blood transfusion. The present invention can also reflect the donee's physical condition before a blood transfusion, for example, whether the donee is an AIDS patient or infected by AIDS, whether the donee is a hepatitis patient or carries hepatitis virus, or whether the donee received blood or blood products before, whether the donee is infected by diseases.

In an example embodiment, the present invention can request or require the signature of a relative of the donee (patient) or the person approved for the blood transfusion (i.e., acknowledge that the blood test report presented by the hospital before a blood transfusion is authentic and reliable and/or agree that the blood transfused is safe or otherwise comes from a legal national blood collection or supply center instead of the hospital or an illegal blood collection institution).

In an example embodiment, the system of the present invention terminal equipment is deployed in five places: blood collecting vehicle (blood collecting station, or plasma collecting station), a clinical laboratory of a blood center (for initial test and re-testing), a composition analysis room, a blood storeroom of the blood center (such as a blood release window, storeroom for blood test, or storeroom of acceptable blood), and a blood bank of a hospital (such as a clinical laboratory).

The blood center may have a computer room for completely recording the workflow of the blood center and the content of blood information terminals in hospital.

The workflow of blood center can include, physical examination, blood collection (blood donation), blood sampling for composition analysis, blood testing (initial testing and final testing), determination of blood type and quantity of acceptable blood, and transport of blood to a blood storeroom. After identification and statistics in the system, the blood can be stored in refrigerators at appropriate temperatures, in accordance with the design storage temperatures of blood components. The blood can then be distributed from a blood storeroom.

In the blood storeroom (or blood release window) in blood center, the computer in the storeroom can record the daily information. In an example embodiment, the daily information includes: (1) daily inventory of acceptable blood in the blood center (i.e., blood volume collected every day, total blood volume, volume per blood type, package types per blood type, number of blood bags per package type, and number of units and package types per blood component; (2) blood volume distributed per type per day (as above); (3) destination hospitals; volume per blood type (as above); (4) complete information of blood distributor; (5) complete information of blood taker; (6) a physical inspection report of the donee (patient) before blood transfusion; (7) the appearance of the blood distributed (i.e., whether there is any blood leakage, fatty blood, or blood coagulation); (8) blood-taking time and blood-release time; (9) whether there is official stamp of the blood-using unit on the blood-taking form, whether the official stamp is clear, who requests for blood; (10) the amount of blood is returned to the blood donors; (11) the percentage of blood-component transfusion in blood volume; (12) discarded blood volume per day, blood volume (ml) per blood type, package types per blood type, instructions as to how to discard the blood, the destination of discarded blood, and who discards the blood, who approves (signs), and the signature of a department director.

In an example embodiment, required information about the blood-receiving terminal is recorded onto electronic tag. This information can include, by way of example, the name of the blood taker, the name of the blood mixer, the name of transfusion operator, the name of the donee, (the blood test report taken before the blood transfusion, the signature provided to acknowledge authenticity, reliability, and legality of the hospital's test report, and the signature time). This information can also include, or be used to determine or ascertain the following: whether transfusion reaction may occur during or after blood transfusion; the measures that should be taken if a reaction occurs; the result of the reaction; if the patient is receiving a blood transfusion is the first time; when the blood transfusion was performed for the first time; the volume of blood volume that was transfused; the blood type used; whether any blood product was transfused or injected; the name of the product; whether the doner is infected by AIDS or other infectious diseases; a blood-test report compiled in three months after the blood transfusion; whether the donee is infected by AIDS or other viruses due to the blood transfusion; whether the donee is permitted to use blood free of charge and, (if so, the volume of "free blood" the donee is permitted to use); whether the donee uses blood at his/her own expense or at the public's expense; the percentage in the total blood volume of blood transfused; the monthly/annual blood volume transfused (the percentage of blood component transfusion in total blood volume); and characteristics of the medical entity performing the transfusion.

In an example embodiment, the present invention records an/or monitors blood-specific information. This information can include or be used to determine, by way of example, the following: the monthly/annual blood volume used by a donee in a blood center (as percentage of blood composition transfusion in total blood volume); whether the percentage meets the blood composition transfusion percentage specified by the state; the difference between the percentages; quantitative comparison with the data from the previous year (by percent volume); the number of blood donors; the volume of blood donated; the percentage in the total blood volume of the blood donated; characteristics of the discarded blood (such as fat content, blood coagulation, blood leakage from inner/outer blood bag, hemoglobin levels). A percentage of all patients infected by viruses or carrying viruses (including AIDS patients) and other conditions related to blood donation prevention or deferral due to non-conformance to governmental test standards can also be ascertained.

In an exemplary embodiment, the present invention can monitor medical faults, medical accidents, and fatal medical accidents per month/year in blood centers, transfusion reactions, and treating schemes.

Medical faults include, for example, wrong blood type, fatty blood, blood coagulation, expired blood, blood leakage, blood damaged due to improper storage methods (such as a wrong refrigerator temperature), and artificial blood is found not to have been transported out of the blood center (blood station).

Medical accidents may occur when blood is transported to the hospital and is found in the hospital before it is transfused to the donees.

Fatal medical accidents may occur when blood has been transfused to the patients and death occurs as, for example, a consequence of mishandling.

Information relating to transfusion reactions may include the number of patients, the percentage in the total number of patients, causes, reactions, handling, and recovery of the patient.

Terminal equipment in blood storeroom in blood center includes an embedded computer, POS or embedded computer, and a code reader. The recorded data in the terminal equipment in the blood storeroom can be identical to the data specified for computer room in blood center;

The recorded data in the terminal equipment in blood bank or clinical laboratory in hospital can be identical to the data specified for the computer room in the blood center.

Blood donation certificate has forgery-prevention design, such as, for example, retina recognition. The blood sources of special blood types (e.g., RH negative) can be shared across the country over a nationwide tracking network information system. The system can use a 2KBit encrypted electronic tag 22, such as an electronic tag 22 from TI company (USA).

In an example embodiment, the present invention utilized a nationwide network. The nationwide network can include a federal blood information network of blood centers in all states, a state information network of blood centers, local municipal or county blood centers in cities, including blood stations, blood storage centers, and blood information networks in hospitals. With existing communication technology, a nationwide network can be implemented, so that public health officials can master the information of blood sources, blood quality, and percentage of blood composition transfusion, etc. across the country at any time. Blood donation certificates can be produced under governmental supervision. Certificates can be numbered uniformly across the country. Current forgery-prevention techniques discourage forgery. Certificate guarantees the blood donor's benefit and national interest, fully reflects the protection and card to the blood donors, and promotes safety of blood donation and medical usage of blood and blood products.

The tracking function of the present invention can decrease "leakage rate" and protect the physical health of medical staffs. For example, the present invention can be used so that AIDS patients, virus carriers, and/or hepatitis C patients are permanently prohibited from donating blood. Specifically, if an infected person is found at any blood collecting point, the case will be reported across the country and the person's information will be marked specially, so that when the person tries to donate again, he/she will be stopped immediately, and the medical staffs will be warned for health protection. Additional features may include tracking such persons with satellite positioning systems and encouraging such persons to seek medical treatment.

The present invention may be sued to identify sources of viral infection (AIDS, hepatitis C, etc.) and thereby reduce the incidence of further infection. For example, the World Health Organization (the WHO) warned last year that the male/female ratio of people infected by AIDS reached 5.5: 4.5. This ratio indicates an AIDS trend of spreading among populations since there is still no cure for many infectious diseases such as AIDS, it is important to detect and control the sources of these diseases, protect healthy medical blood sources, and screen out people infected by or carrying AIDS or hepatitis C virus or a yearly basis in order to improve the quality of blood collected in blood centers (such as blood stations, blood storage centers), promote high blood quality from donors, compensate for the "leakage ratio" problem that persists due to limitations in existing blood testing techniques, and provide an effective platform for the detection, control, and treatment of AIDS, hepatitis C, and syphilis diseases as early as possible.

In an example embodiment, the blood donation certificates of blood donors of special blood types and RH Negative blood will be specially marked, so as to protect the blood source better, utilize the blood in a planned way, and facilitate the overall management and allocation of blood across the country, (such as when fatal natural and man-made disasters occurs). When disaster strikes, help can therefore be coordinated nationwide. The present invention can also facilitate the rescue of people involved in foreign affairs better (e.g., RH Negative blood type).

In an example embodiment, the present invention can create an archive for return visit by a donor after receiving a blood transfusion.

Currently, creating such an archive is difficult because, for example, there is no scientific or sophisticated technical system in place. The automatic identification system of the present invention can greatly influence the development and consummation of medical blood transfusion, blood transfusion safety, and measurement of blood component transfusion.

In accordance with the present invention, the quality of blood products can be ensured by controlling the raw material of blood products. For example, information can be logged regarding whether the plasma is obtained from a good source, whether the plasma is supplied from a blood collecting or supply unit certificated by the state, and the volume and quality of the plasma supplied. In this way, "virus infected" plasma (such as plasma infected by AIDS or hepatitis C) can be identified, and high quality blood products can be ensured. In addition, once the blood product with a specific code is found in question, the cause can be ascertained quickly, so as to minimize the spreading of infectious diseases. Though the codes are displayed on electronic tags, their actual meaning may be restricted to selected health or government officials.

The labor intensity of the medical staffs can be reduced and their efficiency can be improved with the identification techniques of the present invention. After the RFID system is applied in the blood center, multiple bags of blood can be checked at the same time through reader/writer (gate-type antenna), and the computer will handle them and display and print the result automatically. The system is much superior to barcode scanning technique in accuracy, forgery prevention performance, and rapidity. The system can thereby reduce labor intensity of the staffs and improve efficiency.

Readers/writers are available in handheld fixed types, depending on the demand and location of application. The working frequency can be approximately 13.56MHz (high frequency), though other frequencies may be used without departing from the spirit or scope of the present invention. In addition, some information can be written as required. For example, the hospital can set the readers/writers to record the blood test information before blood transfusion and the blood transfusion information of each donee on tag.

The reader/writer sends signals at a preset frequency via antenna. When tag enters into the magnetic field, it produces an induced current and thereby obtains energy and sends its coding information. Reader/writer reads the information, decodes it, and sends the decoded information to a computer for processing.

RFID tags are generally classified into passive tags or active tags. Active tags have battery for power supply, feature longer effective access ranges, are a larger size, and have a higher cost then passive tags. Passive tags generally obtain energy required for operation from the magnetic field produced by a reader/writer, have a lower cost, feature longer service life, have shorter effective access range, and are smaller and lighter than active tags. The working frequency of the RFID system is generally the emitting frequency that is used by reader/writer. The frequency may be emitted in various ranges: a low frequency of 30-300KHz, a high frequency of 3MHz-30MHz, and an ultra high frequency of 300MHz-3GHz. In an example embodiment, the low frequency is 125KHz or 134.2KHz and the high frequency is 13.56MHz.

Compared to barcode technique, a widely used automatic identification technique, the RFID technique of the present invention has several advantages. The advantages include the following:
1) a light source is unnecessary and external material can be penetrated to read data; 2) longer service life and ability to function in harsh environments; 3) ability to be embedded in or attached to products of different types and in different shapes; 4) a longer effective access range; 5) write and read data more quickly; 6) an ability to dynamically modify the content of tag; 7) an ability to handle multiple tags at the same time; 8) tag data access protected by password; 9) an ability to track objects to which RFID tags are attached by means of satellite positioning system; 10) a uniqueness of tags that make tags difficult to forge.

The tracking function of the present invention can decrease "leakage rate" and protect the physical health of medical staffs. For example, the present invention can be used so that AIDS patients, virus carriers, and/or hepatitis C patients are permanently prohibited from donating blood. Specifically, if an infected person is found at any blood collecting point, the case will be reported across the country and the person's information will be marked specially, so that when the person tries to donate again, he/she will be stopped immediately, and the medical staffs will be warned for health protection. Additional features may include tracking such persons with satellite positioning systems and encouraging such persons to seek medical treatment.

The present invention may be sued to identify sources of viral infection (AIDS, hepatitis C, etc.) and thereby reduce the incidence of further infection. For example, the World Health Organization (the WHO) warned last year that the male/female ratio of people infected by AIDS reached 5.5: 4.5. This ratio indicates an AIDS trend of spreading among populations since there is still no cure for many infectious diseases such as AIDS, it is important to detect and control the sources of these diseases, protect healthy medical blood sources, and screen out people infected by or carrying AIDS or hepatitis C virus or a yearly basis in order to improve the quality of blood collected in blood centers (such as blood stations, blood storage centers), promote high blood quality from donors, compensate for the "leakage ratio" problem that persists due to limitations in existing blood testing techniques, and provide an effective platform for the detection, control, and treatment of AIDS, hepatitis C, and syphilis diseases as early as possible.

In an example embodiment, the blood donation certificates of blood donors of special blood types and RH Negative blood will be specially marked, so as to protect the blood source better, utilize the blood in a planned way, and facilitate the overall management and allocation of blood across the country, (such as when fatal natural and man-made disasters occurs). When disaster strikes, help can therefore be coordinated nationwide.

The present invention can also facilitate the rescue of people involved in foreign affairs better (e.g., RH Negative blood type).

In an example embodiment, the present invention can create an archive for return visit by a donor after receiving a blood transfusion. Currently, creating such an archive is difficult because, for example, there is no scientific or sophisticated technical system in place. The automatic identification system of the present invention can greatly influence the development and consummation of medical blood transfusion, blood transfusion safety, and measurement of blood component transfusion.

In accordance with the present invention, the quality of blood products can be ensured by controlling the raw material of blood products. For example, information can be logged regarding whether the plasma is obtained from a good source, whether the plasma is supplied from a blood collecting or supply unit certificated by the state, and the volume and quality of the plasma supplied. In this way, "virus infected" plasma (such as plasma infected by AIDS or hepatitis C) can be identified, and high quality blood products can be ensured. In addition, once the blood product with a specific code is found in question, the cause can be ascertained quickly, so as to minimize the spreading of infectious diseases. Though the codes are displayed on electronic tags, their actual meaning may be restricted to selected health or government officials.

After the RFID system is applied in the blood center, several tens bags of blood can be loaded into a basket or box and checked at the same time through the reader, and the computer will handle them and display and print the result automatically. The system is much superior to barcode scanning technique in accuracy, forgery prevention performance, and rapidity. In addition, it can reduce labor intensity of the staffs and improve working efficiency greatly.

While the present invention has been illustrated and described with reference to some preferred embodiments, the present invention is not limited to these. Those skilled in the art should recognize that various variations and modifications can be made without departing from the spirit and scope of the present invention as defined by the accompanying claims.

## Claims

1. The application of Radio Frequency Identification (RFID) technique in the workflow of a blood center (blood station) or medical institutions such as clinical laboratory in hospital, China Bone Marrow Bank, Sperm Bank, or Ovum Bank, introducing a nationwide Customer Relationship Management (CRM) network (e.g., blood management information system) composed of a RFID system, a computer database system, and a computer information network;
wherein, in the workflow of the blood center (blood station) or medical institutions such as clinical laboratory in hospital, China Bone Marrow Bank, Sperm Bank, or Ovum Bank, the information transfer between the electronic tags attached to things (e.g., blood bags) and files (e.g., blood donation certificates) and the reader/writer is through radio waves, all information of the people, things, and files involved in the workflow is read/written by the electronic tag reader/writer through the computer information network into an embedded electronic identification system and stored in the back office management system (computer database system).

2. The system of RFID technique in the workflow of blood center or medical institution as in claim 1, wherein, said system comprises terminal equipment and RFID equipment; said RFID equipment comprises electronic tag reader/writer and antenna; said terminal equipment is connected to said reader/writer through a data bus; the antenna of said reader/writer sends signals at a pre-set frequency; when said RFID tag enters into the magnetic field, it produces induced current and thereby obtains energy and sends its coding information; said reader/writer obtains the information, decodes it, and then sends the decoded information to the terminal equipment for data exchange, so as to identify the RFID tag that has unique electronic ID and is attached to blood bag to identify the target object and the RFID blood donation certificate that contains a variety of information; said RFID tag is featured with long storage life (at least 10 years) and tearing resistance.

3. The system of RFID technique in the workflow of blood center or medical institution as in claim 1, wherein, said electronic tag reader/writer is of handheld or fixed type, works at 13.56MHz (high frequency), and employs an embedded electronic identification device; said terminal equipment and said electronic tag reader/writer are deployed in the computer equipment room, blood storeroom or at blood release window in blood center, or the blood bank or clinical laboratory in hospital.

4. The system of RFID technique in the workflow of blood center or medical institution as in claim 1, wherein, the RFID blood donation certificate has a sign for special blood type or RH Negative blood type.

5. The system of RFID technique in the workflow of blood center or medical institution as in claim 1, wherein, the computer with electronic tag reader/writer is connected to the computer information network through an existing public line or wireless data link or private line or wireless data link and supports information encryption.

6. An application of RFID system in the workflow of blood center or medical institution, comprising a network customer relationship management information system composed of RFID system, computer database system, and computer information network; wherein, in the workflow of blood center or medical institution, the information transfer between the integrated circuit in the electronic tag attached to things or files of blood or biological products (i.e., electronic tag attached to things or files) and the reader/writer is through radio wave, all relevant information ranging from identity information to biological product information in each procedure of the blood collecting and supply workflow is read/written by the computer with electronic tag reader/writer and through the computer information network into the service management information system.

7. The application of RFID system in the workflow of blood center or medical institution as in claim 6, wherein, in the workflow of blood center or medical institution, the information in the electronic tag attached to things or files of biological products (e.g., blood) is encrypted, and identity authentication or access authority control is carried out for the person who accesses that information.

8. The application of RFID system in the workflow of blood center or medical institution as in claim 6, wherein, all useful information in the service processing workflow is stored by the RFID system to create a variety of categorized, sorted, and statistical information and provide information search and service management capability.

9. The application of RFID system in the workflow of blood center or medical institution as in claim 6, wherein, the entire workflow of blood center comprising: collecting blood from a blood donor -sub-packing components for test-- testing (including initial testing and re-testing) -- acceptable or unacceptable - transporting the acceptable blood to blood storeroom - transporting the blood to the blood bank in hospital and matching type - transfusing the blood to donee -- logging the destination of blood bags.

10. The application of RFID system in the workflow of a blood center or a medical institution as in claim 6, wherein, the equipment or system employs embedded electronic identification technique, executes special functions, and is controlled by the internal computer, and the software is solidified software (i.e., firmware).
